(19) Europäisches Patentamt
European Patent Office
Office européen des brevets

(11) Veröffentlichungsnummer: **0 369 216 B1**

(12) **EUROPÄISCHE PATENTSCHRIFT**

(45) Veröffentlichungstag der Patentschrift: **16.06.93**

(51) Int. Cl.$^5$: **A61K 31/58**

(21) Anmeldenummer: **89119837.6**

(22) Anmeldetag: **25.10.89**

# (54) Arzneimittelzubereitung aus Esterderivaten des Hecogenins und dessen Verwendung zur Behandlung von benigner Prostatahyperplasie.

(30) Priorität: **15.11.88 DE 3838716**

(43) Veröffentlichungstag der Anmeldung:
**23.05.90 Patentblatt 90/21**

(45) Bekanntmachung des Hinweises auf die Patenterteilung:
**16.06.93 Patentblatt 93/24**

(84) Benannte Vertragsstaaten:
**AT CH DE ES FR GB GR LI LU NL SE**

(56) Entgegenhaltungen:
**EP-A- 0 285 383**
**DE-A- 2 759 171**
**FR-A- 2 201 893**
**GB-A- 2 024 624**

(73) Patentinhaber: **Boots Pharma GmbH**
**Oberer Weberberg 11**
**W-8884 Höchstädt/Donau(DE)**

(72) Erfinder: **Streber, August S.**
**Mühlweg 2**
**W-8909 Aichen(DE)**

(74) Vertreter: **Hansen, Bernd, Dr.rer.nat. et al**
**Hoffmann, Eitle & Partner Patentanwälte Arabellastrasse 4 Postfach 81 04 20**
**W-8000 München 81 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 369 216 B1

## Beschreibung

Die Erfindung betrifft eine Arzneimittelzübereitung auf der Basis von Hecogeninderivaten und deren Verwendung.

Das zu den steroidalen Sapogeninen zählende Hecogenin (Merck Index S. 667) wird in der glykosidisch gebundenen Form, in der das H-Atom der OH-Gruppe durch eine Mono-oder Disaccharidgruppe ersetzt ist, zur Hemmung von Entzündungen beschrieben (DE-OS 29 26 463, DE-OS 27 59 171). Dabei können die Mono- oder Disaccharidgruppen teilweise oder ganz mit Monocarbonsäuren verestert sein. Eine besonders bevorzugte Verbindung aus der Reihe der Glykosidderivate des Hecogenins ist dabei Hecogenin-$\beta$-D-glucosid und/oder dessen Ester. Die entzündungshemmende Aktivität der oben genannten Verbindungen beruht auf einer Inhibierung der Prostaglandinsynthese. Die Glykosidderivate des Hecogenins werden insbesondere für die Behandlung von begleitenden Entzündungsvorgängen, auch bei benigner Prostatahyperplasie beschrieben. Das Behandlungsziel liegt in der die benigne Prostatahyperplasie reaktiv und konsekutiv begleitenden Prostatitis. Die genannten Hecogeninderivate sind jedoch nicht dazu bestimmt, die benigne Prostatahyperplasie kausal durch Hemmung des Zellwachstums und durch Reduzieren des Volumens der vergrößerten Prostata zu behandeln.

Für die Linderung und Heilung von benigner Prostatahyperplasie sind bislang Brennesselwurzelextrakte der Urtica dioica, der Urtica urens und/oder deren Hybriden vorgesehen (vgl. Bundesanzeiger Nr. 173, S. 13286). Wie viele Naturprodukte können diese Heilmittel Qualitätsschwankungen unterliegen und sie weisen einen geringeren Wirkstoffgehalt auf, so daß im Vergleich zu reinen, synthetischen Wirksubstanzen eine vergleichsweise hohe Dosierung erforderlich ist.

Die Aufgabe der vorliegenden Erfindung besteht darin, eine Arzneimittelzübereitung zu schaffen. Diese soll insbesondere bei notwendiger Therapie im Urogenitalbereich einsetzbar sein. Bevorzugt soll sie nicht nur die Enzündungserscheinungen der benignen Prostatahyperplasie lindern, sondern auch auf Grund einer Hemmwirkung des Zellwachstums eine Verbesserung bzw. Heilung der benignen Prostatahyperplasie erreichen können.

Die Aufgabe wird erfindungsgemäß durch eine Arzneimittelzübereitung gelöst, die als pharmazeutisch wirksame Substanz Esterderivate des Hecogenins der allgemeinen Formel

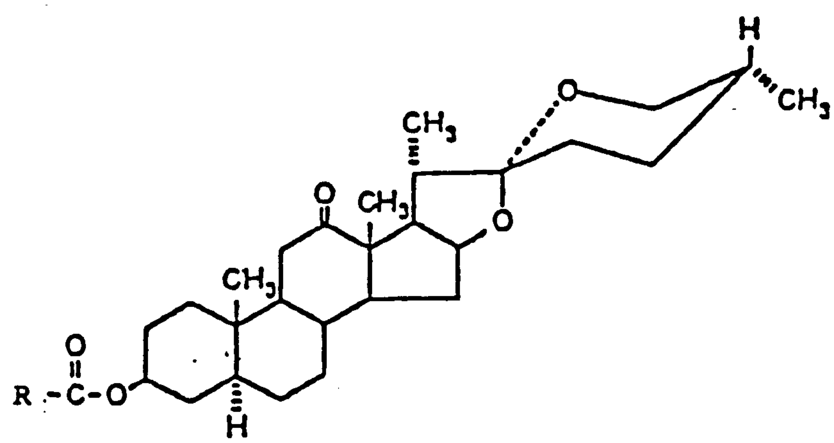

in der R eine Ethyl-, Propyl- oder Butylgruppe darstellt, und pharmazeutisch verträgliche Träger enthält.

Die Synthese der Verbindungen der allgemeinen Formel (I) kann in üblicher Weise durch Verestern des Hecogenins mit den entsprechenden Säuren erfolgen. Sie können in an sich bekannter Weise mit Hilfe geeigneter, pharmazeutisch verträglicher Träger zu Pulvern, Tabletten, Kapseln, Dragées, und Pillen für die orale Verabreichung und zu Emulsionen oder Lösungen für eine Injection oder Infusion verarbeitet werden. Die geeignete Dosierungsmenge liegt vorzugsweise im Bereich von 3 bis 30 mg/kg Körpergewicht Wirksubstanz pro Tag, und die Verabreichung erfolgt vorteilhafterweise als Dauertherapie oder auch als vierteljährliche Kurbehandlung mit entsprechenden Pausen.

Die Erfindung umfaßt schließlich die Verwendung von Hecogeninderivaten der allgemeinen Formel, in der R ein $C_{1-4}$-Alkyl darstellt, und deren pharmazeutisch verträglichen Trägern, zur Herstellung eines Medikamentes Behandlung von benigner Prostatahyperplasie bei Mensch und Tier.

Versuch 1: In-vivo Untersuchungen des Prostatavolumens

In einer Versuchsreihe verabreichte man unter benigner Prostatahyperplasie leidenden Hunden die erfindungsgemässe Arzneimittelzübereitung oral in Form von Dragées über einen Zeitraum von 100 Tagen. Die tägliche Dosis betrug dabei ein Dragée je 10 kg Körpergewicht, das als Wirksubstanz 5,0 mg Hecogeninacetat enthielt. Nach der Behandlungsdauer wurde der Rückgung des Prostatavolumens sonographisch kontrolliert.

| Versuchsgruppe: | Rückgang des Prostatavolumens (%) nach der Behandlungsdauer von 100 Tagen |
|---|---|
| Hund Nr. 1 | 17,9 |
| Hund Nr. 2 | 60,1 |
| Hund Nr. 3 | 31,1 |
| Hund Nr. 4 | 4,9 |
| Hund Nr. 5 | 13,4 |
| Hund Nr. 6 | 87,8 |
| Hund Nr. 7 | 61,9 |
| | Mittelwert: 39,6 |

Die obigen Ergebnisse zeigen deutlich die hohe Wirksamkeit der erfindungsgemässen Arzneimittelzübereitung.

Versuch 2: Untersuchung der akuten Toxizität von Hecogeninacetat

Die Substanz Hecogeninacetat wurde auf akute orale und akute intraperitoneale Toxizität an der NMRI-Maus, der SPRD-Ratte und dem NZW-Kaninchen untersucht.

Die Mäuse (beiderlei Geschlechts, 23-25 g) und die Ratten (beiderlei Geschlechts, 130-150 g) entstammten eine kontrollierten SPF-Zucht (Züchter: IWF GmbH, 8192 Geretsried-Gelting), die Kaninchen (1,9 kg) stammten aus der Zucht: Luise Brendt, 8710 Kitzingen.

Die Versuchstiere wurden konventionell in künstlich belüfteten Räumen (Sterilluft) bei 20-21 °C und 50-61 % relativer Luftfeuchtigkeit unter Kunstlicht gehalten. Der Hell-Dunkel-Wechsel erfolgte jeweils nach 12 Stunden, der Luftwechsel ca. 10 mal pro Stunde. Die Fütterung geschah ad libitum mit Altromin Standard Diät, die Tränkung ad libitum mit Leitungswasser, das teilenthärtet war und periodisch mikrobiologisch kontrolliert wurde.

Die Haltung der Mäuse und Ratten erfolgte in Makrolenkäfigen Typ III, die Kaninchenhaltung in Ebeco Ganzdrahtkäfigen.

Die Vorversuchshaltung der Tiere dauerte 1 Woche.

1. Akute orale Toxizitätsprüfung:

| | |
|---|---|
| Versuchstiere: | NMRI-Maus |
| Dosis: | 25 mg bis 2,0 g/kg Körpergewicht |
| Tierzahl/Dosis: | n = 10 (5 m. + 5 w.) |
| Applikationsvolumen: | 40,0 ml/kg Körpergewicht einheitlich |
| Versuchstiere: | SPRD-Ratte |
| Dosis: | 25 mg bis 2,0 g/kg Körpergewicht |
| Tierzahl/Dosis: | n = 10 (5 m. + 5 w.) |
| Applikationsvolumen | 40,0 ml/kg Körpergewicht einheitlich |

2. Akute intraperitoneale Toxizitätsprüfung:

| | |
|---|---|
| Versuchstiere: | NMRI-Maus |
| Dosis: | 0,1 mg bis 50 mg/kg Körpergewicht |
| Tierzahl/Dosis: | n = 10 (5 m. + 5 w.) |
| Applikationsvolumen: | 40,0 ml/kg Körpergewicht einheitlich |
| Versuchstiere: | NZW-Kanninchen |
| Dosis: | 0,5 bis 2,0 g/kg Körpergewicht |
| Tierzahl/Dosis: | n = 2 |
| Applikationsvolumen: | 40,0 ml/kg Körpergewicht einheitlich. |

Ergebnisse:

Die Versuchsmäuse und -ratten vertrugen in dem Test zur akuten oralen Toxizitätsprüfung die verabreichten Dosen symptomlos. Die maximal verabreichte Menge entspricht etwa 140 g beim Menschen. Auch bei dem akuten, intraperitonealen Toxizitätstests traten sowohl bei den Mäusen wie auch bei den Kanninchen mit den verabreichten Dosen keine Symptome auf. Die maximal verabreichten Mengen entsprechen beim Menschen etwa 35 g bzw. 140 g. Bei den einzelnen Versuchen konnte eine $LD_{50}$ jeweils bis zur Obergrenze der angegebenen Dosierung nicht ermittelt werden. Die Substanz erwies sich bei den eingesetzten Tieren nur wenig toxisch und ist gemäß der Gefährlichkeitsmerkmale-Verordnung (BGBI 1487) als mindergiftig einzustufen. Mit akuten Vergiftungen innerhalb der untersuchten Dosen ist nicht zu rechnen. Danach sind auch beim Menschen Vergiftungen bei einmalig oral verabreichten Mengen bis zu etwa 10 g nicht zu erwarten.

Mit den erfindungsgemässen Arzneimittelzübereitungen ist ein Produkt auf der Basis von Esterderivaten des Hecogenins geschaffen, das auf Grund einer Hemmung des Zellwachstums die kausale Behandlung von benigner Prostatahyperplasie ermöglicht, das in sehr kleinen Wirkmengen eingesetzt werden kann und das als untoxisch zu betrachten ist.

Im folgenden wird beispielhaft die Herstellung einer Arzneimittelzübereitung mit Hecogeninacetat als Wirkstoff beschrieben.

Beispiel: Arzneimittelherstellung

Die folgenden Substanzen werden in einer Kugelmühle vermischt und anschließend mit einem 8 mm Drageestempel gepresst:

| | |
|---|---|
| Hecogeninacetat | 10,00 mg |
| Lactose | 134,00 mg |
| Ca-carboxymethylcellulose | 30,00 mg |
| Avicel | 10,00 mg |
| Talcum | 15,00 mg |
| Mg-stearat | 1,00 mg |
| | 200,00 mg |

**Patentansprüche**

**Patentansprüche für folgende Vertragsstaaten : AT, CH, DE, FR, GB, LI, LU, NL, SE**

**1.** Arzneimittelzubereitung dadurch **gekennzeichnet,** daß sie als pharmazeutisch wirksame Substanz Esterderivate des Hecogenins der allgemeinen Formel

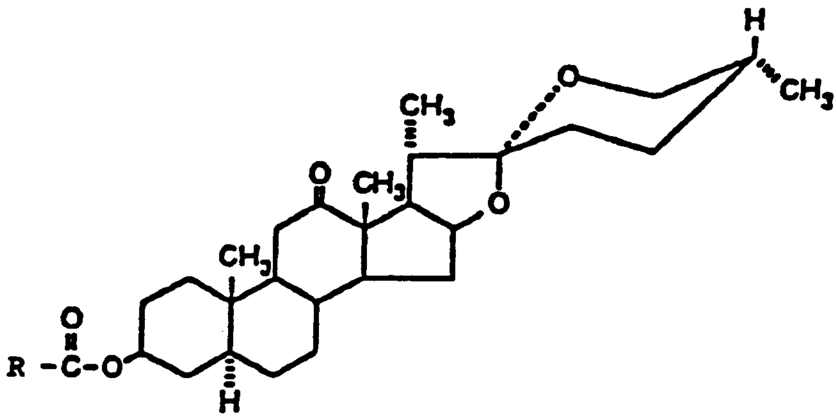

in der R eine Ethyl-, Propyl- oder Butylgruppe ist, und pharmazeutisch verträgliche Träger enthält.

2. Verwendung von Esterderivaten des Hecogenins der allgemeinen Formel

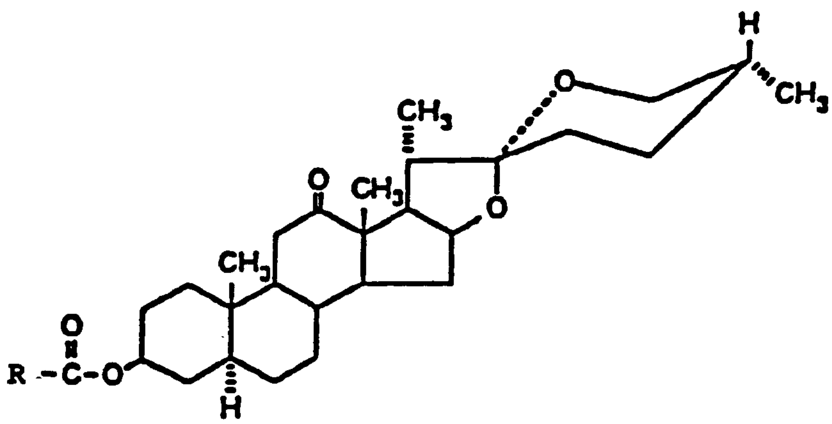

in der R ein $C_{1-4}$-Alkyl darstellt, und deren pharmazeutisch verträglichen Trägern, zur Herstellung eines Medikamentes zur Behandlung benigner Prostatahyperplasie bei Mensch und Tier.

**Patentansprüche für folgende Vertragsstaaten : ES, GR**

1. Verfahren zur Herstellung einer Arzneimittelzubereitung, **gekennzeichnet** durch Vermischen von Esterderivaten des Hecogenins der allgemeinen Formel

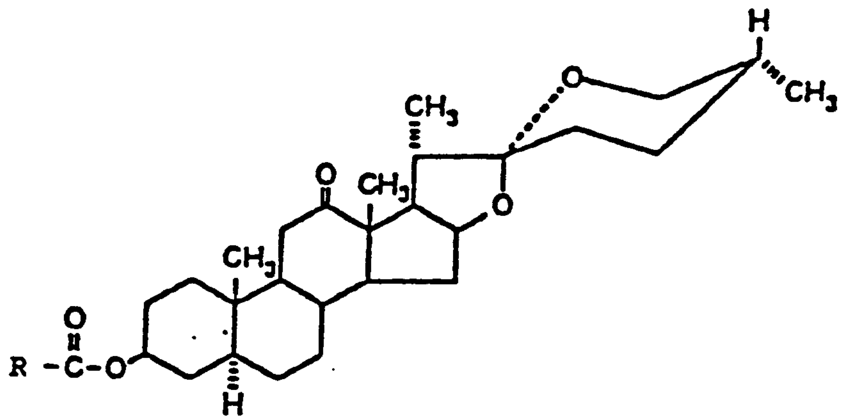

in der R eine Ethyl-, Propyl- oder Butylgruppe ist, als pharmazeutisch wirksame Substanz in einer Dosierungsmenge von 3 bis 30 mg/kg Körpergewicht pro Tag mit geeigneten pharmazeutisch verträglichen Trägern.

**2.** Verwendung von Esterderivaten des Hecogenins der allgemeinen Formel

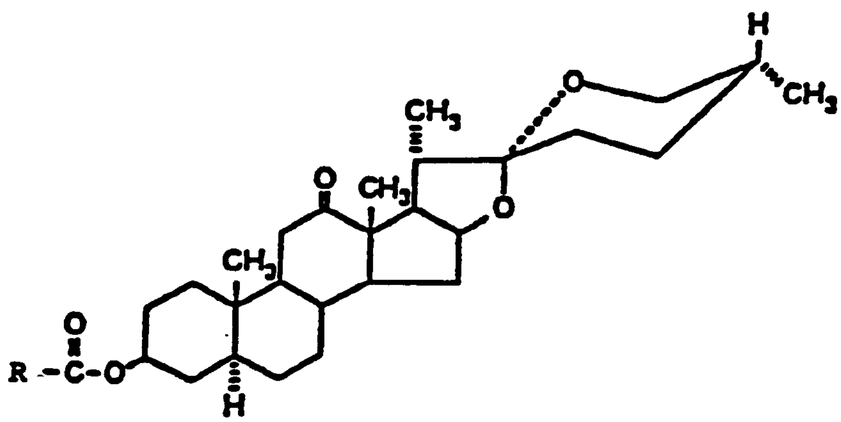

in der R ein $C_{1-4}$-Alkyl darstellt, zur Herstellung eines Medikamentes zur Behandlung von benigner Prostatahyperplasie bei Mensch und Tier.

**Claims**

**Claims for the following Contracting States : AT, CH, DE, FR, GB, LI, LU, NL, SE**

**1.** Medicinal preparation, characterised in that it contains ester derivatives of hecogenin of the general formula

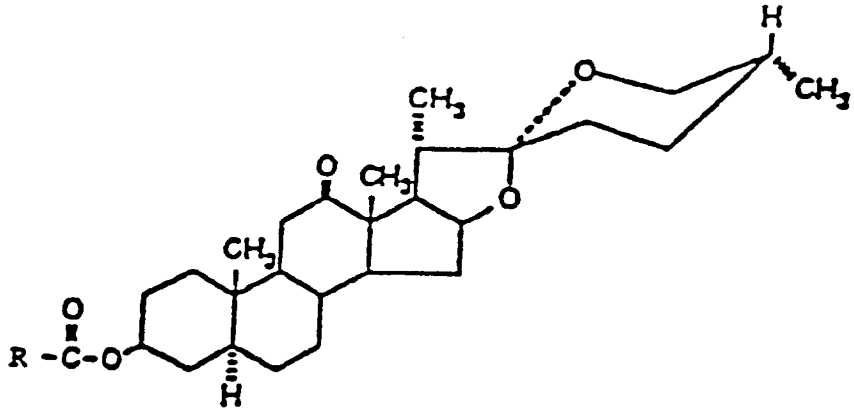

in which R is an ethyl, propyl or butyl group, as pharmaceutically active substance, and pharmaceutically acceptable excipients.

**2.** Use of ester derivatives of hecogenin of the general formula

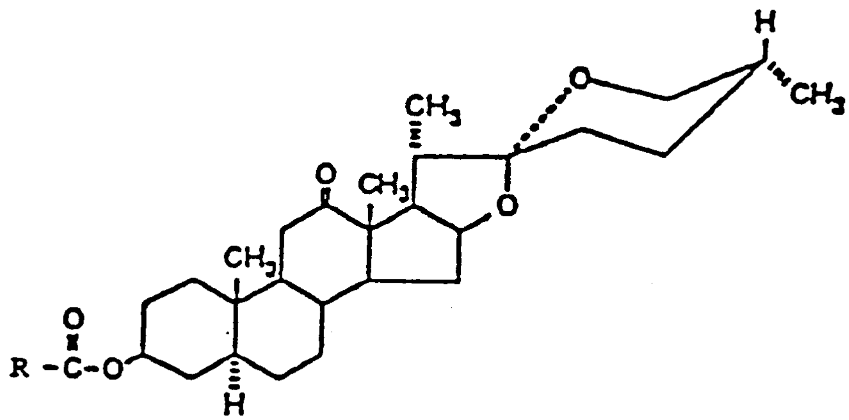

in which R is $C_{1-4}$-alkyl, and their pharmaceutically acceptable excipients, for producing a medicament for treating benign prostatic hyperplasia in humans and animals.

**Claims for the following Contracting States : ES, GR**

**1.** Process for producing a medicinal preparation, characterised by mixing ester derivatives of hecogenin of the general formula

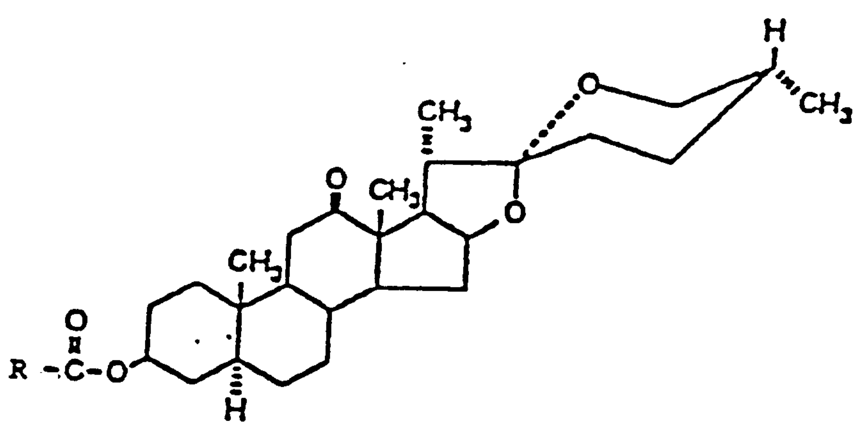

in which R is an ethyl, propyl or butyl group, as pharmaceutically active substance in a dose quantity of 3 to 30 mg/kg of body weight per day, with suitable pharmaceutically acceptable excipients.

**2.** Use of ester derivatives of hecogenin of the general formula

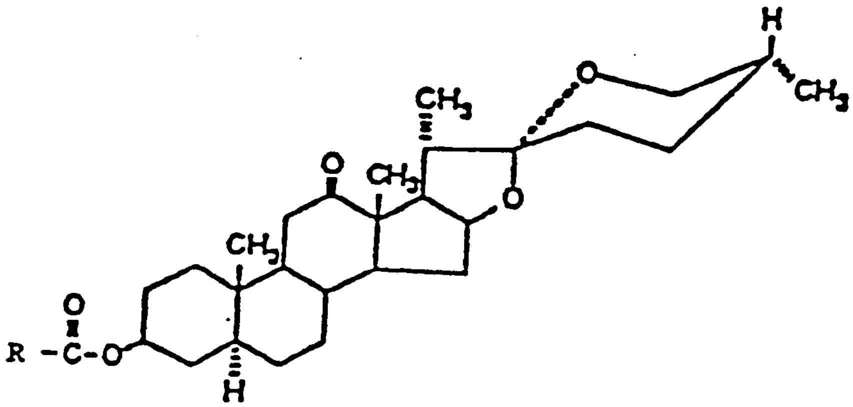

in which R is $C_{1-4}$-alkyl, for producing a medicament for treating benign prostatic hyperplasia in humans and animals.

**Revendications**

**Revendications pour les Etats contractants suivants : AT, CH, DE, FR, GB, LI, LU, NL, SE**

**1.** Préparation médicamenteuse, caractérisée en ce qu'elle contient comme substance pharmaceutiquement active des esters de l'hécogénine répondant à la formule générale :

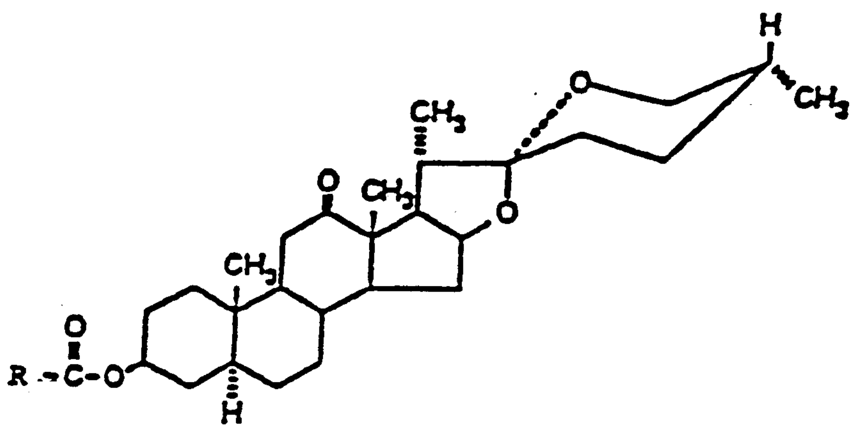

dans laquelle R est un groupe éthyle, propyle ou butyle, et un support pharmaceutiquement acceptable.

2. Utilisation d'esters de l'hécogénine répondant à la formule générale :

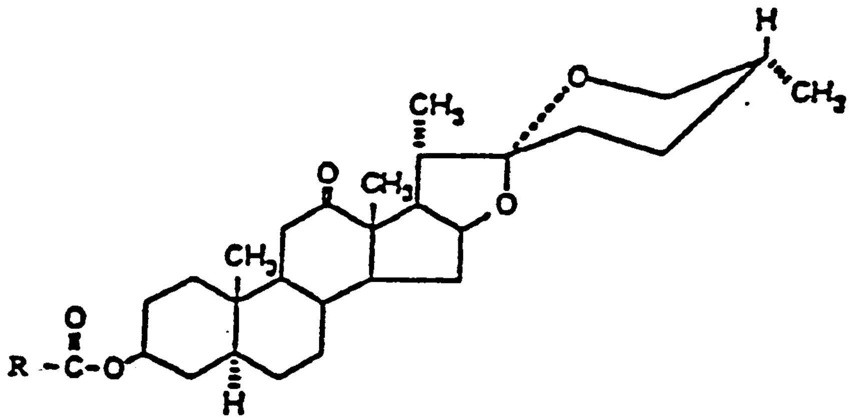

dans laquelle R représente un groupe alxyle en $C_1$ à $C_4$, et de leur support pharmaceutiquement acceptable, pour la fabrication d'un médicament pour le traitement de l'hyperplasie prostatique bénigne chez l'homme et l'animal.

**Revendications pour les Etats contractants suivants : ES, GR**

1. Procédé de confection d'une préparation médicamenteuse, caractérisé en ce qu'on mélange des esters de l'hécogénine répondant à la formule générale :

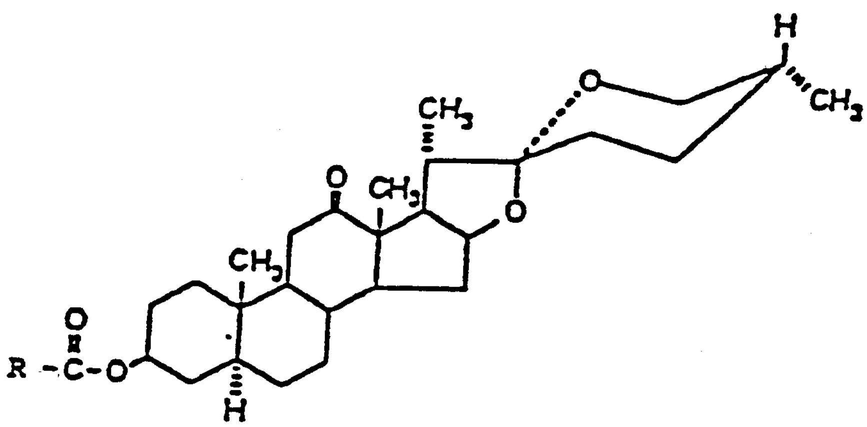

dans laquelle R est un groupe éthyle, propyle ou butyle, comme substance pharmaceutiquement active, à la dose de 3 à 30 mg/kg de poids corporel par jour, avec des supports pharmaceuciquement acceptables.

**2.** Utilisation d'esters de l'hécogénine répondant à la formule générale :

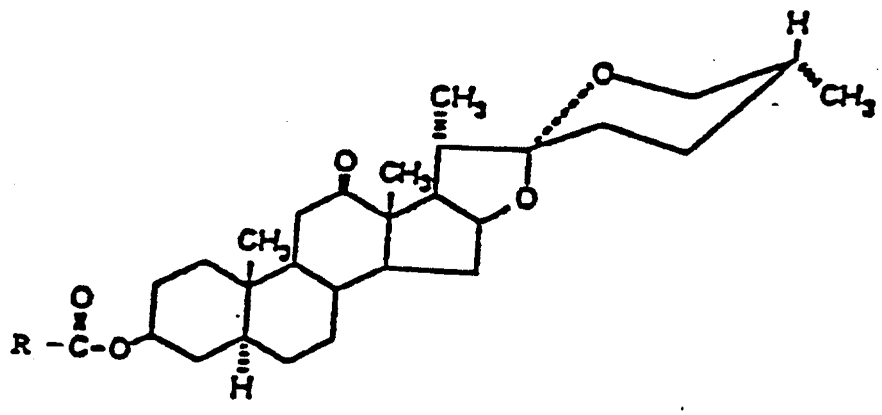

dans laquelle R représente un groupe alkyle en $C_1$ à $C_4$, pour la fabrication d'un médicament pour le traitement de l'hyperplasie prostatique bénigne chez l'homme et l'animal.